# NEUE EUROPÄISCHE PATENTSCHRIFT

(11) **EP 0 158 249 B2**
(45) Veröffentlichungstag und Bekanntmachung des Hinweises auf die Entscheidung über den Einspruch: **16.03.1994**
(45) Hinweis auf die Patenterteilung: 29.08.1990
(21) Anmeldenummer: 85103855.4
(22) Anmeldetag: 30.03.1985
(51) Int. Cl.: A61K 7/50

(54) **Medizinische Ölbäder**
Medicinal oil bath
Bain d'huile médicinal

(30) Priorität: 11.04.1984 DE 3413563
(43) Veröffentlichungstag der Anmeldung: 16.10.1985
(73) Patentinhaber: MERCK PATENT GmbH, D-64271 Darmstadt (DE)
(72) Erfinder: Franke, Rolf, D-2057 Reinbek (DE); Schmersahl, Peter, Dr., D-2000 Willinghusen b. Hamburg (DE)

(56) Entgegenhaltungen:
- EP-A- 0 018 463
- EP-A- 0 020 867
- EP-A- 0 092 256
- EP-A- 0 158 108
- FR-A- 1 086 004
- FR-A- 1 199 691
- FR-A- 1 526 808
- FR-A- 2 097 333
- FR-A- 2 252 403
- FR-A- 2 515 034
- H. JANISTYN: "Handbuch der Kosmetika und Riechstoffe", Band 3: "Die Körperpflegemittel", 2. Auflage, Seiten 453-461, 1973, Dr. Alfred Hüthig Verlag, Heidelberg, DE
- Präparate-Verzeichnis der Ichthyol-Gesellschaft Cordes, Hermani & Co. vom 01/12/83
- Rote Liste 1983
- Römpps Chemielexikon, 8. Auflage, Bd 2,1981 S.1213
- Ullmanns Encyklopädie der technischen Chemie Bd 22, 1982 S.489-491

## Beschreibung

Die Erfindung betrifft medizinische Ölbäder.

Medizinische Ölbäder werden bekanntlich von Patienten mit trockener Haut angewendet, um den beim Waschen mit Seifen oder Syndets eintretenden Verlust an Hautfett zu vermeiden. Sie bestehen im wesentlichen aus für den Waschvorgang nötigen Emulgatoren, denen aber gleichzeitig "rückfettende" Öle zugesetzt sind (vgl. z.B. Rote Liste 1984 Nr. 25009, 25016, 25038, 25081-83).

Eine häufige Begleiterscheinung bei Hautkrankheiten mit trockener Haut ist der Juckreiz, der oft auch nach Anwendung medizinischer Ölbäder bestehen bleibt.

Als nächster Stand der Technik ist ein Produkt namens Ölbad Cordes® (Rote Liste 1984 Nr. 25016) anzusehen, welches aber ebenfalls keine zufriedenstellende juckreizstillende Wirkung aufweist.

Der Erfindung lag die Aufgabe zugrunde, ein medizinisches Ölbad aufzufinden, das selbst juckreizstillend wirkt.

Es wurde gefunden, daß der Zusatz eines der üblichen Lokalanästhetika für diesen Zweck nicht erforderlich ist, daß es vielmehr genügt, dem Ölbad zwei oder drei Emulgatoren aus der Reihe der Fettalkoholpolyglykolether der allgemeinen Formel

C₁₂H₂₅-(O-CH₂CH₂)ₙ-OH (I),

worin n eine ganze Zahl zwischen 2 und 15, vorzugsweise zwischen 4 und 12 bedeutet. Besonders bevorzugt sind Laurylpolyglykolether (I, R = C₁₂H₂₅), die z.B. unter der Bezeichnung Laureth n wie Laureth 4, Laureth 9, Laureth 10 im Handel sind: es handelt sich in der Regel Gemische, wobei die Ziffer n die durchschnittliche Zahl der Ethylenoxid-Gruppen anzeigt. Laureth 9 ist besonders bevorzugt; ein Gemisch, dessen Zusammensetzung durch die Formel

C₁₂H₂₅-(OCH₂CH₂)_{ca.9}-OH

beschrieben werden kann, ist Polidocanol, das ebenfalls besonders bevorzugt ist.

Die lokalanästhetischen und antipruriginösen Wirkungen einiger dieser Fettalkoholpolyglykolether, z.B. diejenigen des Polidocanols, sind an sich bekannt. Es ist jedoch überraschend, daß diese Wirkungen auch noch in der Anwendungskonzentration des Präparats, nämlich einer Verdünnung mit Wasser im Verhältnis von ca. 1:5000, auftreten; bei dieser Anwendungskonzentration liegen die wirksamen Fettalkoholpolyglykolether nur in Konzentrationen von etwa 0,0025 bis 0,015% im Badewasser vor. Es war nicht anzunehmen, daß die Stoffe in dieser geringen Konzentration noch wirksam sein könnten.

Gegenstand der Erfindung ist ein medizinisches Ölbad, enthaltend etwa 68-95 Gewichtsprozent eines oder mehrerer physiologisch unbedenklicher Öle und etwa 30-5 Gewichtsprozent mehrerer physiologisch unbedenklicher Emulgatoren sowie gewünschtenfalls weitere Hilfsstoffe, dadurch gekennzeichnet, daß es als Emulgator zwei oder drei Fettalkoholpolyglykolether der angegebenen Formel I enthält.

Als physiologisch unbedenkliche Öle kommen alle galenisch üblichen vegetablischen und mineralischen Öle in Betracht. Besonders bevorzugt sind die erstgenannten, vor allem Sojaöl, ferner Erdnußöl, unter den mineralischen Ölen ist Paraffinöl bevorzugt.

Die erfindungsgemäßen Ölbäder enthalten etwa 70-95, vorzugsweise etwa 80-85 Gewichtsprozent eines oder mehrerer der genannten Öle und etwa 30-5, vorzugsweise etwa 20-10 Gewichtsprozent Emulgatoren. Das Gemisch der verwendeten Emulgatoren zeigt vorzugsweise HLB-Werte (hydrophile-lipophile balance) im Bereich von etwa 12 bis 14.

Die erfindungsgemäßen Ölbäder können weitere Bestandteile enthalten, beispielsweise; Konservierungsmittel; Antioxydantien wie BHT (butyliertes Hydroxytoluol) oder BHA (butyliertes Hydroxyanisol); Lösungsvermittler, z.B. Alkohole wie Isopropanol oder 2-Octyl-1-dodecanol, Stabilisatoren wie Hectorit.

Die lokalanästhetische Wirkung der erfindungsgemäßen Ölbäder kann z.B. mittels Corneareflex-Prüfung am Kaninchenauge in Anlehnung an die Methode von Regnier ermittelt werden (vgl. M. J. Regnier, C. R. Acad. Sci. Paris *177*, 558-560, 1923; Th. Eckert u. E. Wachtel, Arzneimittelforschung/Drug. Res. *33*, 98-100, 1983), ferner auch an der menschlichen Unterlippe und Zunge mit Hilfe eines elektrischen Reizgebergeräts (Lippenreiz- und Zungenreiztest).

Die in den nachstehenden Formulierungsbeispielen den einzelnen Bestandteilen vorangestellten Zahlen bedeuten Gewichtsteile.

### Beispiel 1

- 5: Laureth 4
- 5: Laureth 9
- 5: Laureth 10
- 83,75: Sojaöl
- 1: Parfüm
- 0,25 BHT: (butyliertes Hydroxytoluol)

### Beispiel 2

- 25: Laureth 4
- 5: Laureth 9
- 68,75: Sojaöl
- 1: Parfüm
- 0,25: BHT

### Beispiel 3

- 5: Laureth 4
- 5: Laureth 9
- 5: Laureth 10
- 82,95: Sojaöl
- 1: Parfüm
- 0,05: BHT
- 0,9: flüssiges Paraffin
- 0,1: Hectorit

### Beispiel 4

- 4,5: Laureth 4
- 4,5: Laureth 9
- 4,5: Laureth 10
- 45: Sojaöl
- 31,45: Erdnußöl
- 1: Parfüm
- 0,05: BHT
- 4,5: Isopropanol
- 4,5: 2-Octyl-1-dodecanol

## Patentansprüche

1. Medizinisches Ölbad mit antipruriginöser Wirkung enthaltend etwa 68-95 Gewichtsprozent eines oder mehrerer physiologisch unbedenklicher Öle und etwa 30-5 Gewichtsprozent mehrerer physiologisch unbedenklicher Emulgatoren sowie gewünschtenfalls weitere Hilfsstoffe, dadurch gekennzeichnet, daß der Emulgator-Anteil aus zwei oder drei Fettalkoholpolyglykolethern der allgemeinen Formel I
C₁₂H₂₅-(O-CH₂CH₂)ₙ-OH (I)
worin:
n eine ganze Zahl zwischen 2 und 15 bedeutet, besteht.

2. Medizinisches Ölbad gemäß Anspruch 1, enthaltend 70 bis 85 Gewichtsprozent eines oder zweier Öle ausgewahlt aus der Gruppe bestehend aus Sojaöl und Erdnußöl, 30 bis 10 Gewichtsprozent zweier oder dreier Fettalkoholpolyglykolether der Formel I nach Anspruch 1, worin n eine ganze Zahl zwischen 4 und 10 bedeutet, und weiteren Hilfsstoffen, ausgewählt aus der Gruppe bestehend aus 2-Octyl-1-dodecanol, Isopropanol, flüssigem Paraffin, Parfüm, butyliertem Hydroxytoluol und Hectorit.

3. Medizinisches Ölbad gemäß Anspruch 2, enthaltend 83,75 Gewichtsprozent Sojaöl, 15 Gewichtsprozent dreier Fettalkoholpolyglykolether der Formel I nach Anspruch 1 zu gleichen Teilen, worin n 4, 9 und 10 bedeutet, 1 Gewichtsprozent Parfüm und 0,25 Gewichtsprozent butyliertes Hydroxytoluol.

4. Medizinisches Ölbad gemäß Anspruch 2, enthaltend 45 Gewichtsprozent Sojaöl, 31,45 Gewichtsprozent Erdnußöl, 13,5 Gewichtsprozent dreier Fettalkoholpolyglykolether der Formel I nach Anspruch 1 zu gleichen Teilen, worin n 4, 9 und 10 bedeutet, 4,5 Gewichtsprozent Isopropanol, 4,5 Gewichtsprozent 2-Octyl-1-dodecanol, 1 Gewichtsprozent Parfüm und 0,05 Gewichtsprozent butyliertes Hydroxytoluol.

5. Medizinisches Ölbad gemäß Anspruch 2, enthaltend 68,75 Gewichtsprozent Sojaöl, 25 Gewichtsprozent eines Fettalkoholpolyglykolethers der Formel I nach Anspruch 1, worin n 4 bedeutet, 5 Gewichtsprozent eines Fettalkoholpolyglykolethers der Formel I nach Anspruch 1, worin n 9 bedeutet, 1 Gewichtsprozent Parfüm und 0,25 Gewichtsprozent butyliertes Hydroxytoluol.

6. Medizinisches Ölbad gemäß Anspruch 2, enthaltend 82,95 Gewichtsprozent Sojaöl, 0,9 Gewichtsprozent flüssiges Paraffin, 15 Gewichtsprozent dreier Fettalkoholpolyglykolether der Formel I nach Anspruch 1 zu gleichen Teilen, worin n 4, 9 und 10 bedeutet, 0,1 Gewichtsprozent Hectorit, 1 Gewichtsprozent Parfum und 0,05 Gewichtsprozent butyliertes Hydroxytoluol.

## Claims

1. Medicinal oil bath with antipruritic effect containing about 68-95 per cent by weight of one or more physiologically acceptable oils and about 30-5 per cent by weight of several physiologically acceptable emulsifiers and, if desired, other auxiliaries, characterized in that the emulsifier content comprises two or three fatty alcohol polyglycol ethers of the general formula I
C₁₂H₂₅-(O-CH₂CH2)ₙ-OH I,
in which
n is an integer between 2 and 15.

2. Medicinal oil bath according to Claim 1, containing 70 to 85 per cent by weight of one or two oils selected from the group comprising soya oil and arachis oil, 30 to 10 per cent by weight of two or three fatty alcohol polyglycol ethers of the formula I according to Claim 1, in which n is an integer between 4 and 10, and other auxiliaries selected from the group comprising 2-octyl-1-dodecanol, isopropanol, liquid paraffin, perfume, butylated hydroxytoluene and hectorite.

3. Medicinal oil bath according to Claim 2, containing 83.75 per cent by weight of soya oil, 15 per cent by weight of three fatty alcohol polyglycol ethers of the formula I according to Claim 1 in equal parts, in which n is 4, 9 and 10, 1 per cent by weight of perfume and 0.25 per cent by weight of butylated hydroxytoluene.

4. Medicinal oil bath according to Claim 2, containing 45 per cent by weight of soya oil, 31.45 per cent by weight of arachis oil, 13.5 per cent by weight of three fatty alcohol polyglycol ethers of the formula I according to Claim 1 in equal parts, in which n is 4, 9 and 10, 4.5 per cent by weight of isopropanol, 4.5 per cent by weight of 2-octyl-1-dodecanol, 1 per cent by weight of perfume and 0.05 per cent by weight of butylated hydroxytoluene.

5. Medicinal oil bath according to Claim 2, containing 68.75 per cent by weight of soya oil, 25 per cent by weight of a fatty alcohol polyglycol ether of the formula I according to Claim 1, in which n is 4, 5 per cent by weight of a fatty alcohol polyglycol ether of the formula I according to Claim 1, in which n is 9, 1 per cent by weight of perfume and 0.25 per cent by weight of butylated hydroxytoluene.

6. Medicinal oil bath according to Claim 2, containing 82.95 per cent by weight of soya oil, 0.9 per cent by weight of liquid paraffin, 15 per cent by weight of three fatty alcohol polyglycol ethers of the formula I according to Claim 1 in equal parts, in which n is 4, 9 and 10, 0.1 per cent by weight of hectorite, 1 per cent by weight of perfume and 0.05 per cent by weight of butylated hydroxytoluene.

## Revendications

1. Bain d'huile médicinal à action antiprurigineuse contenant environ 68-95% en poids d'une ou plusieurs huiles physiologiquement acceptables et environ 30-35% en poids de plusieurs émulsifiants physiologiquement acceptables ainsi que, si on le désire, d'autres additifs, caractérisé en ce que la partie émulsifiant se compose de deux ou trois polyglycoléthers d'alcools gras de formule générale I
C₁₂H₂₅-(O-CH₂CH₂)ₙ-OH (I)
dans laquelle
n représente un nombre entier compris entre 2 et 15.

2. Bain d'huile médicinal selon la revendication 1, contenant de 70 à 85% en poids d'une ou de deux huiles choisies dans le groupe constitué par l'huile de soja et l'huile d'arachide, de 30 à 10% en poids de deux ou trois polyglycoléthers d'alcools gras de formule I selon la revendication 1, où n représente un nombre entier compris entre 4 et 10, et d'autres additifs, choisis dans le groupe constitué par le 2-octyl-1-dodécanol, l'isopropanol, la paraffine liquide, un parfum, l'hydroxytoluène butylé et l'hectorite.

3. Bain d'huile médicinal selon la revendication 2, contenant 83,75% en poids d'huile de soja, 15% en poids de trois polyglycoléthers d'alcools gras de formule I selon la revendication 1 en parties égales, où n vaut 4, 9 et 10, 1% en poids de parfum et 0,25% en poids d'hydroxytoluène butylé.

4. Bain d'huile médicinal selon la revendication 2, contenant 45% en poids d'huile de soja, 31,45% en poids d'huile d'arachide, 13,5% en poids de trois polyglycoléthers d'alcools gras de formule I selon la revendication 1 en parties égales, où n vaut 4, 9 et 10, 4,5% en poids d'isopropanol, 4,5% en poids de 2-octyl-l-dodécanol, 1% en poids de parfum et 0,05% en poids d'hydroxytoluène butylé.

5. Bain d'huile médicinal selon la revendication 2, contenant 68,75% en poids d'huile de soja, 25% en poids d'un polyglycoléther d'alcool gras de formule I selon la revendication 1, où n vaut 4, 5% en poids d'un polyglycoléther d'alcool gras de formule I selon la revendication 1, où n vaut 9, 1% en poids de parfum et 0,25% en poids d'hydroxytoluène butylé.

6. Bain d'huile médicinal selon la revendication 2, contenant 82,95% en poids d'huile de soja, 0,9% en poids de paraffine liquide, 15% en poids de trois polyglycoléthers d'alcools gras de formule I selon la revendication 1 en parties égales, où n vaut 4, 9 et 10, 0,1% en poids d'hectorite, 1% en poids de parfum et 0,05% en poids d'hydroxytoluène butylé.
